# EUROPÄISCHE PATENTSCHRIFT

(11) **EP 4 003 209 B1**
(45) Veröffentlichungstag und Bekanntmachung des Hinweises auf die Patenterteilung: **02.04.2025**
(21) Anmeldenummer: 20747352.1
(22) Anmeldetag: 21.07.2020
(51) Int. Cl.: A61B 90/00

(54) **IMPLANTIERBARER MARKER**
IMPLANTABLE MARKER
MARQUEUR IMPLANTABLE

(30) Priorität: 24.07.2019 DE 102019210963
(43) Veröffentlichungstag der Anmeldung: 01.06.2022
(73) Patentinhaber: Bip Biomed. Instrumente & Produkte GmbH, 82299 Türkenfeld (DE)
(72) Erfinder: HESKE, Thomas, 82284 Grafrath (DE)
(74) Vertreter: Rösler, Uwe
(86) Internationale Anmeldenummer: PCT/EP2020/070548
(87) Internationale Veröffentlichungsnummer: WO 2021/013831

(56) Entgegenhaltungen:
- EP-A1- 1 105 053
- EP-A1- 1 105 053
- WO-A1-99/43268
- WO-A1-99/43268
- WO-A2-2007/094001
- WO-A2-2007/094001
- US-A1- 2014 221 828
- US-A1- 2014 221 828
- ANONYMOUS: "Kugelschreiber - Wikipedia", 13 July 2019 (2019-07-13), de.wikipedia.org, XP055742811, Retrieved from the Internet <URL:https://de.wikipedia.org/w/index.php?title=Kugelschreiber&oldid=190392853> [retrieved on 20201022]
- ANONYMOUS: "Kugelschreiber - Wikipedia", 13 July 2019 (2019-07-13), de.wikipedia.org, XP055742811, Retrieved from the Internet <URL:https://de.wikipedia.org/w/index.php?title=Kugelschreiber&oldid=190392853> [retrieved on 20201022]

## Beschreibung

### Technisches Gebiet

Die Erfindung bezieht sich einen implantierbarer Marker zum Markieren eines tierischen oder menschlichen intrakorporalen Gewebebereiches, der wenigstens einen aus biokompatiblen Material gefertigten Strang aufweist, der eine im Wege eines Form-gebenden Verfahrens eingeprägte Raumform besitzt, die der Strang nach einer ihm durch äußeren mechanischen Zwang aufgezwungenen kompaktierten Raumform nach Wegfall des mechanischen Zwangs annimmt.

### Stand der Technik

Gattungsgemäße implantierbare Marker werden zur Kennzeichnung von Tumoren in menschlichen oder tierischen Weichteilgewebe eingesetzt. Bspw. wird häufig nach einer Brustbiopsie ein Marker mittels einer Kanüle an den Ort der Gewebeentnahme eingeführt und nach Erreichen der gewünschten Lage mittels eines Mandrins aus der Kanüle distalseitig ausgestoßen. Der intrakorporal platzierte Marker verbleibt ortsfest und bietet für einen Arzt die Möglichkeit einen zu therapierenden und/oder zu diagnostizierenden Gewebebereich mit Hilfe eines bildgebenden Verfahrens, vorzugsweise anhand von Ultraschallaufnahmen zu orten und langfristig zu beobachten.

Der Druckschrift US 6,053,925 ist hierzu ein Gewebemarker für menschliches Gewebe zu entnehmen, der zwei miteinander verdrillte Drähte aus Formgedächtnismetall aufweist. Der Marker, der in einem zu markierenden Gewebebereich möglichst ortsunveränderlich zu platzieren ist, weist hierzu eine ring- oder spulenförmige Form auf.

Der Druckschrift US 2005/0059888 A1 ist ein Marker zu entnehmen, der den Ort eines intrakorporal verbrachten biologischen Absorberkörpers markiert. Der Marker besteht aus einem im Wege der Mammographie, Radiologie und Ultraschalluntersuchung detektierbaren Material, bspw. aus einem Draht, der an dem Absorberkörper angebracht ist.

Der Druckschrift US 2001/0023322 A1 ist eine kanülenartige Positioniereinheit für einen intrakorporal einbringbaren Marker zu entnehmen. Der Marker besteht aus einem Formgedächtnismetalldraht, der sich nach intrakorporaler Positionierung zumindest an der Drahtspitze ring- oder spulenartig zu Zwecken einer festen Positionierung in einem zu markierenden Gewebebereich verformt.

Der Druckschrift WO 2007/094001 A2 ist ein Marker zu entnehmen, der aus einem gewundenen Draht besteht, der in verschiedenen Bereichen unterschiedliche Steigungen aufweist.

Schließlich ist der Druckschrift EP 1 871 266 B1 ein gattungsgemäßer Marker für menschliches oder tierisches Gewebe zu entnehmen, der aus einem vorprogrammierbaren Material, vorzugsweise einer Nickel-Titan-Legierung, ringförmig gefertigt ist, der sich nach einer ihm aufgezwungenen Längserstreckung nach Freigabe in die vorprogrammierte Ringform zurückbildet.

Allen bekannten gattungsgemäßen Markern, so insbesondere der vorstehend erläuterte ringförmige Marken, stellen aufgrund ihrer Materialeigenschaften Ultraschallreflektoren dar, doch zeichnen sich die bekannten Marker in Abhängigkeit der Beschallungsrichtung, längs der die Ultraschallwellen mit dem Marker in Wechselwirkung treten, anhand der Ultraschallbildaufnahmen unterschiedlich stark ab. Trifft ein sich zumeist fächerförmig ausbreitendes Ultraschallwellenfeld auf einen vorbezeichneten Ringmarker, dessen Ringebene orthogonal zur Fächerebene der sich ausbreitenden Ultraschallwellen orientiert ist, so zeichnen sich lediglich zwei punktartige Ultraschallreflexionssignale ab, die bei Verschwenken des Ultraschallfeldes relativ zum ringförmigen Marker die Ringkontur wiedergeben. Ist das fächerförmige Ultraschallwellenfeld hingegen parallel zur Ringebene orientiert, so bilden mögliche Reflexionssignale am Ultraschallbild eine Linie mit einer Länge, die dem Durchmesser des Ringmarkers entspricht.

Es bedarf daher großer Erfahrung, derartige an sich bekannte Marker, deren Dimensionierung im Bereich weniger Millimeter liegen, räumlich und ortsaufgelöst innerhalb des biologischen Gewebeumfeldes eindeutig zu erkennen.

### Darstellung der Erfindung

Der Erfindung liegt die Aufgabe zugrunde, einen implantierbaren Marker zum Markieren eines tierischen oder menschlichen intrakorporalen Gewebebereiches, der wenigstens einen aus biokompatiblen Material gefertigten Strang aufweist, der eine im Wege eines Form-gebenden Verfahrens eingeprägte Raumform besitzt, die der Strang nach einer ihm durch äußeren mechanischen Zwang aufgezwungenen kompaktierten Raumform nach Wegfall des mechanischen Zwangs annimmt, derart weiterzubilden, dass die Sichtbarkeit des implantierten Markers für einen Arzt während einer Ultraschalluntersuchung erheblich verbessert sein soll. Insbesondere soll sich der Marker unabhängig von den mit dem Ultraschallkopf zu beschallenden Richtungen durch eine weitgehend isotrope hohe Ultraschallreflektivität auszeichnen, so dass eine für den Arzt visuell eindeutige und leichte Ortung des Markers mit Hilfe eines Ultraschall-basierten Bildgebungsverfahren möglich sein soll.

Die Lösung der der Erfindung zugrundeliegenden Aufgabe ist im Anspruch 1 angegeben. Den Erfindungsgedanken vorteilhaft weiterbildende Merkmale sind Gegenstand der Unteransprüche sowie der Beschreibung unter Bezugnahme auf die Ausführungsbeispiele in Verbindung mit den Figuren zu entnehmen.

Lösungsgemäß zeichnet sich der implantierbare Marker mit den Merkmalen des Oberbegriffes des Anspruches 1 dadurch aus, dass die dem Strang eingeprägte Raumform wenigstens drei feststehende Strang-Augen umfasst, die jeweils durch wenigstens eine helikale Wicklung des Stranges gebildet sind und deren Form und/oder räumliche Relativlage zueinander in der durch den äußeren mechanischen Zwang aufgezwungenen kompaktierten Raumform sowie in der nach Wegfall des mechanischen Zwangs eingeprägten Raumform unterschiedlich sind.

Der Begriff "feststehendes Strang-Auge" ist aus der Knotenkunde entlehnt und stellt eine einfache geometrische Form dar, die sich durch das Bilden eines Kreises längs des Stranges auszeichnet. Hierbei wird der Strang wenigstens einmalig helikal gewickelt und weist eine helikale Steigung auf, bei der sich der Strang im Überlappungsbereich der Wicklung berührt. Im Unterschied zu einer in sich geschlossenen Ringform stellt sich ein feststehendes Auge bei seitlicher Ultraschallwellenbeschallung nicht als gerade Linie mit gleichbleibender Liniendicke, sondern keil- oder doppelkeilförmig dar, deren maximale Keilbreite bei einer einfachen helikalen Wicklung der doppelten Strangbreite entspricht. Entsprechend deutlicher bildet sich ein Ultraschallsignal bei seitlicher Beschallung eines aus mehreren helikalen Wicklungen zusammengesetztes feststehende Strang-Auge aus.

Der lösungsgemäße implantierbare Marker verfügt über drei oder mehr Strang-Augen, die längs des Strangs durch jeweils eine helikale Wicklung des Strangs gebildet sind. Nicht notwendigerweise, jedoch in vorteilhafter Form verfügen die wenigstens zwei feststehenden Strang-Augen über gleiche Formen und Dimensionen. Selbstverständlich sind auch von der Kreisform abweichende Augenformen, wie bspw. ovale oder elliptische feststehende Augen denkbar. Auch können sich die Formen und Dimensionen der längs des Stranges angeordneten Strang-Augen voneinander der unterscheiden. Zudem sind die räumliche Lage und Zuordnung der feststehenden Strang-Augen durch deren Abstand zueinander und Orientierung der Ebenen, die den feststehenden Strang-Augen jeweils zuordenbar sind, einheitlich oder individuell wählbar.

In besonderer Weise eignet sich für die Fertigung des Stranges und der Einprägung der lösungsgemäßen Raumform längs des Stranges metallisches Formgedächtnismaterial, das vorzugsweise über ein pseudoelastisches Verhalten verfügt, d. h. der aus dem Formgedächtnismaterial bestehende Draht kehrt in seine im Wege eines Hochtemperaturprozesses eingeprägte Raumform formerhaltend zurück, selbst wenn der Draht unter Aufbringung äußerer Krafteinwirkung elastisch stark verform wird. Besonders geeignete Materialien sind Nickel-Titan ("Nitinol"), Nickel-Titan-Kupfer, Kupfer-Zink, Kupfer-Zink-Aluminium oder Kupfer-Aluminium-Nickel.

In einer möglichen Ausführungsform des lösungsgemäßen implantierbaren Markers weist der Strang drei feststehende Strang-Augen auf, die über einen Strangabschnitt einstückig miteinander verbunden sind. Zum Zwecke der intrakorporalen Verortung wird der Strang durch äußeren mechanischen Zwang in eine kompaktierte Raumform überführt, in der die wenigstens eine helikale Wicklung der Strang-Augen durch längs des Stranges wirkende Zugkräfte vollständig gestreckt werden, d. h. die Wicklungen werden weitgehend vollständig unter Ausbildung eines linearen Stranges elastisch verformt. In dieser kompaktierten Form wird der Strang in eine Hohlkanüle eingebracht, durch die der Strang intrakorporal platzierbar ist.

Das Ausbringen des Stranges erfolgt in an sich bekannter Weise mit Hilfe eines Mandrins. Sobald der in der kompaktierten Raumform befindliche Strang distalseitig aus der Hohlkanüle ausgestoßen ist, nimmt der Strang seine eingeprägte Raumform spontan ein und bildet die drei feststehenden Strang-Augen spontan aus. Je nach eingeprägter Raumform können die Strang-Augen jeweils eine gemeinsame Augenebene aufspannen. Vorzugsweise bietet es sich an, die einzelnen feststehenden Strang-Augen längs des Stranges mit jeweils unterschiedlichen Augen-Ebenen zu orientieren, d. h. längs des Strangabschnittes, der beide feststehenden Strang-Augen miteinander einstückig verbindet, ist eine torsionale, materialinhärente Materialspannung eingeprägt, die beide feststehenden Strang-Augen relative zueinander verdreht orientiert.

Alternativ oder in Kombination mit der vorstehenden Verdrehung beider über einen Strangabschnitt einstückig miteinander verbundenen feststehenden Strang-Augen sieht ein weiteres Ausführungsbeispiel ein Verkippen bzw. Verschwenken beider feststehenden Strang-Augen durch eine lokal eingeprägte Krümmung längs des Strangabschnittes vor, der beide feststehenden Strang-Augen einstückig miteinander verbindet.

In einem weiteren Ausführungsbeispiel sind die wenigstens drei feststehenden Strang-Augen über einen Strangabschnitt einstückig verbunden, so dass sie in der durch den äußeren mechanischen Zwang aufgezwungenen kompaktierten Raumform überlappungsfrei nebeneinander längs einer virtuell Linearachse angeordnet sind. In diesem Zustand werden sie in eine entsprechend dem Augendurchmesser dimensionierte Hohlkanüle eingeschoben und, wie im vorstehenden Fall bereits erläutert, mit Hilfe eines Mandrins distalseitig über die Hohlkanüle innerhalb eines Körpers implantiert. Unmittelbar nach Austritt aus der Hohlkanüle erfolgt eine spontane elastische Verformung des implantierbaren Markers in seine ihm eingeprägte Raumform. Im Falle zweier über einen Strangabschnitt einstückig miteinander verbundener feststehender Strang-Augen können diese ihre initial eingeprägte Form und/oder Größe sowie auch ihre räumliche Lage zueinander individuell einnehmen.

In einer weiteren bevorzugten Ausführungsform sind längs des Stranges wenigsten drei feststehende Strang-Augen ausgebildet, die in der durch den äußeren mechanischen Zwang aufgezwungenen kompaktierten Raumform längs einer virtuellen Linearachse seriell hintereinander angeordnet sind. In dieser kompaktierten Raumform lässt sich der implantierbare Marker durch eine geeignet dimensionierte Hohlkanüle intrakorporal platzieren. Sobald der durch die Hohlkanüle auf den Marker einwirkende, mechanische Zwang durch distales Ausstoßen wegfällt, nimmt der implantierbare Marker seine ihm initial eingeprägte Raumform an, die sich signifikant von der seriellen Anordnung der wenigstens drei feststehenden Strang-Augen längs der virtuellen Linearachse unterscheidet.

Anhand der im Weiteren figürlich dargestellten Ausführungsbeispiele lässt sich der große Variantenreichtum an möglichen Ausgestaltungsformen des lösungsgemäß implantierbaren Markers mit den ihm lösungsgemäß eingeprägten Raumformen illustrieren, die sich durch eine verbesserte Sichtbarkeit aus allen Raumrichtungen mit Hilfe eines ultraschallbasierten Bildgebungsverfahrens auszeichnet.

Neben der die verbesserte Ultraschallwellen-basierte Detektierbarkeit begründenden eingeprägten Raumform des implantierbaren Markers trägt zudem eine bevorzugt längs der Strangoberfläche angebrachte bzw. eingearbeitete Oberflächenstrukturierung zur Erhöhung der Ultraschallwellen-Reflektivität bei, die vorzugsweise in Form einer Oberflächenaufrauhung oder einer stochastischen oder regelmäßigen Anordnung von rillenartigen, nutförmigen und/oder kerbartigen Ausnehmungen an der Strangoberfläche charakterisiert ist.

Alternativ zur Ausbildung des lösungsgemäßen implantierbaren Markers in Form eines aus metallischem Formgedächtnismaterial bestehenden Drahtes und dessen lösungsgemäß eingeprägter Raumform bietet es sich gleichsam an, den Strang aus einem geeignet gewählten alternativen biokompatiblen Material zu fertigen, das über gleiche oder ähnliche Formgedächtniseigenschaften verfügt. Bspw. eignen sich hierzu Formgedächtnispolymere oder Kompositwerkstoffe, die Polymere in Kombination mit Formgedächtnislegierungen vorsehen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnung exemplarisch beschrieben. Es zeigen:
- Fig. 1a: erste Ausführungsform eines nicht erfindungsgemäß ausgebildeten implantierbaren Markers in einer kompaktierten Raumform,
- Fig. 1b, c: Drauf- und Seitenansicht des implantierbaren Markers gemäß Fig. 1a mit eingeprägter Raumform,
- Fig. 2a: zweites Ausführungsbeispiel eines erfindungsgemäßen implantierbaren Markers in kompaktierter Raumform,
- Fig. 2b, c, d: unterschiedliche Betrachtungswinkel auf den implantierbaren Marker gemäß Fig. 2a in der eingeprägten Raumform, sowie
- Fig. 3a - c: verschiedene Strang-Oberflächenstrukturierungen.

### Wege zur Ausführung der Erfindung, gewerbliche Verwendbarkeit

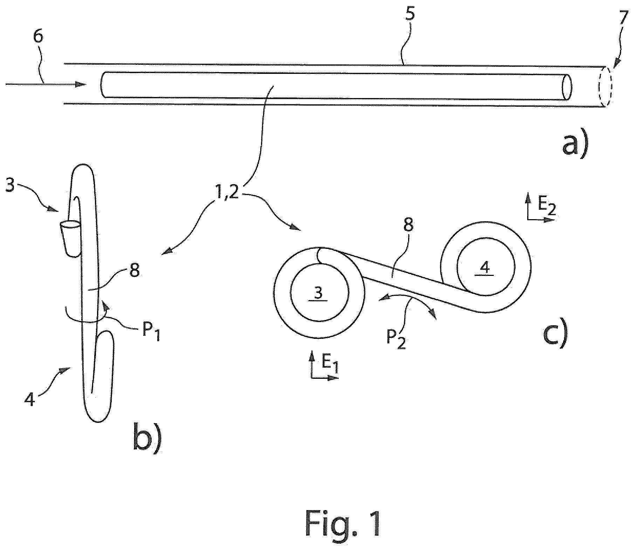

In den Figuren 1a - c ist ein erstes Ausführungsbeispiel für einen lösungsgemäß ausgebildeten implantierbaren Marker illustriert. Die Figuren 1b und c zeigen den Marker 1 in der Drauf- und Seitenansicht, jeweils in seiner dreidimensional eingeprägten Raumform. Der Marker 1 ist aus einem Runddraht 2, vorzugsweise aus Nitinol (NiTi) gefertigt und weist in seiner eingeprägten Raumform zwei feststehende Strang- bzw. Draht-Augen 3, 4 auf. Die Draht-Augen 3, 4 sind jeweils durch eine einlagige Helikalwicklung längs des aus Nitinol bestehenden Drahtes gefertigt. Der Wicklungssinn beider Draht-Augen 3, 4 ist jeweils entgegengesetzt orientiert.

Zum Zwecke der intrakorporalen Verortung des implantierbaren Markers 1 wird der Marker 1 durch einen äußeren mechanischen Zwang in die in Figur 1a dargestellte linear gestreckte, kompaktierte Raumform überführt. Hierzu wird der Nitinoldraht 2 aus seiner in den Figuren 1b und c illustrierten eingeprägten Raumform unter Zugkrafteinwirkung in Drahtlängsrichtung gestreckt und in eine Hohlkanüle 5 eingeführt.

Mit Hilfe eines Mandrins 6 wird der in der kompaktierten Raumform befindliche Draht 2 durch die distale Öffnung 7 der Hohlkanüle 5 geschoben. Unmittelbar nach Austritt des Markers 1 durch die distale Öffnung 7 der Hohlkanüle 5 nimmt der Marker 1 die in den Figuren 1b und c illustrierte eingeprägt Raumform an. Die beiden über einen Drahtabschnitt 8 einstückig miteinander verbundenen, feststehenden Draht-Augen 3, 4 weisen jeweils in der Zeichenebene liegende Augenebenen E1, E2 auf. Selbstverständlich können die zu den feststehenden Drahtaugen 3, 4 zugeordneten Augenebenen E1, E2 auch geneigt bzw. verdreht zueinander orientiert sein. Hierzu gilt es längs des Drahtabschnittes 8 eine entsprechend mechanische materialinhärente Vorspannung vorzusehen, durch die ein Verdrehen beider feststehenden Draht-Augen 3, 4 relativ zueinander initiiert wird, siehe hierzu Pfeildarstellung P1 in Figur 1b. Alternativ zu der vorstehenden Verdrehung oder in Kombination mit der Verdrehung kann längs des Drahtabschnittes 8, der in den Figuren 1b, c geradlinig ausgebildet ist, eine mechanisch vorprogrammierte Drahtkrümmung eingebracht sein, wie dies durch die in Figur 1c ersichtliche Pfeildarstellung P2 angedeutet ist.

Die in Figur 1 dargestellte, dem implantierbaren Marker 1 eingeprägte Raumform, ermöglicht eine leichte und sichere Detektion mit Hilfe eines ultraschallbasierten Bildgebungsverfahren, unabhängig von der räumlichen Orientierung des implantierten Markers 1 zu dem jeweils intrakorporal eingeschallten Ultraschallwellenfeld.

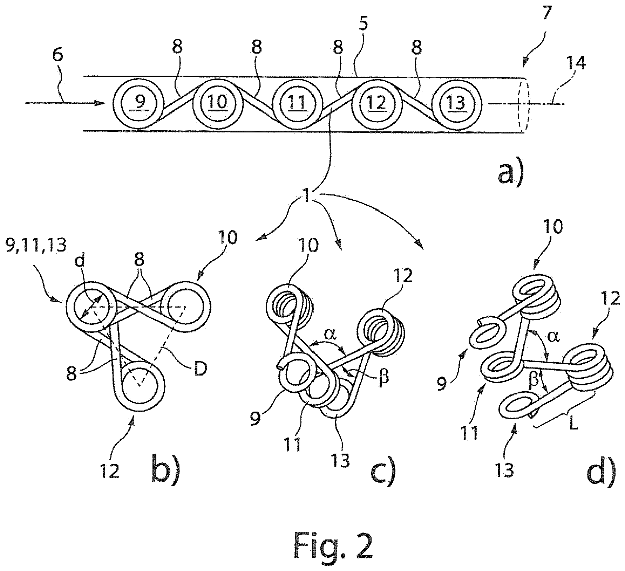

Die Figuren 2a - d illustrieren eine bevorzugte erweiterte Variante zur Ausbildung eines lösungsgemäß implantierbaren Markers 1, dessen eingeprägte Raumform aus unterschiedlichen Betrachtungswinkeln in den Figuren 2b - d dargestellt ist.

Der implantierbare Marker 1 verfügt in diesem Fall über fünf feststehende Strang- bzw. Draht-Augen 9 - 13, die jeweils einstückig, vorzugsweise aus einem Nitinoldraht 2 gefertigt sind. Anhand der Figuren 2b - d, die den implantierbaren Marker 1 auf unterschiedlichen perspektivischen Betrachtungswinkeln zeigen, ist ersichtlich, dass die feststehenden Draht-Augen 10 und 12 jeweils mehr als eine helikale Wicklung vorsehen. Die Anzahl der Wicklungen, aus denen ein feststehendes Draht-Auge gefertigt ist, kann variabel beliebig gewählt werden. Auch kann die Anzahl der Draht-Augen längs des Drahtes 2 sowie deren Abstände und Orientierungen zueinander beliebig gewählt werden.

In Figur 2a ist die kompaktierte Raumform des implantierbaren Markers 1 gezeigt, in der die feststehenden Draht-Augen 9 bis 13 längs einer virtuellen Linearachse 14 unter äußerem mechanischen Zug-Zwang linear angeordnet sind, so dass sie innerhalb einer Hohlkanüle 5 einführbar sind. Sobald der in der kompaktierten Raumform vorliegende Marker 1 mit Hilfe des Mandrins 6 distalseitig aus der Hohlkanüle 5 geschoben worden ist, nimmt der Marker 1 sprunghaft, d. h. spontan die in den Figuren 2b - d illustrierte eingeprägte Raumform an. Hierbei bilden die feststehenden Draht-Augen 9 - 13 gemeinsam mit den Draht-Abschnitten 8 eine dreidimensional bestimmte Struktur bzw. Raumform, die in Wechselwirkung mit einem Ultraschallwellenfeld signifikante Reflexionssignale erzeugt, die auf einem Ultraschallbild visuell signifikant in Erscheinung treten. Zudem vermag der lösungsgemäß ausgebildete implantierbare Marker 1 aufgrund des sprunghaften Übergangs aus der in Figur 2a dargestellten kompaktierten Raumform in die in den Figur 2b - d illustrierte eingeprägte Raumform eine innige und mechanisch stabile Verbindung mit dem den Marker 1 umgebenden biologischen Gewebe einzugehen, so dass eine unerwünschte Migration des implantierten Markers 1 innerhalb des Gewebes von Anfang an ausgeschlossen werden kann.

Nur der Vollständigkeit halber sei darauf hingewiesen, dass abweichend von der in den Figuren 2b - d illustrierten eingeprägten Raumform des implantierbaren Markers 1 auch Verdrehungen oder Verkrümmungen jeweils längs der Drahtabschnitte 8 eingebracht sein können, siehe Figuren 1b , c die Pfeildarstellungen P1, P2.

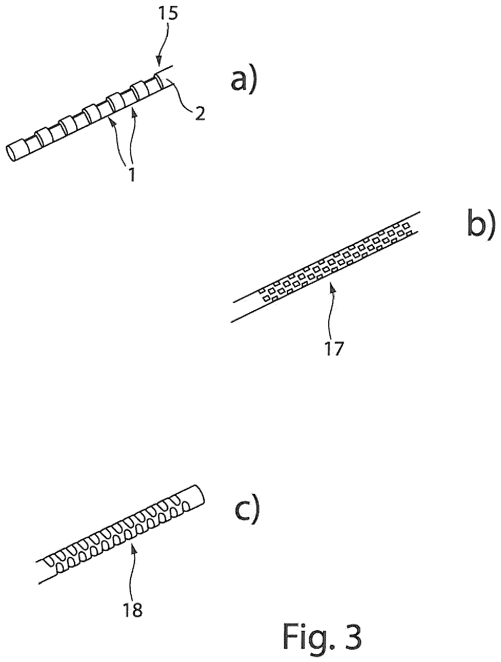

Um die Ultraschallreflektivität des lösungsgemäßen implantierbaren Markers 1 zu verbessern kann die Oberfläche 15 des Stranges bzw. Drahtes 2, aus dem der Marker 1 besteht, strukturiert werden. Mögliche Oberflächenstrukturierungen des Stranges bzw. Drahtes 2 sind in den Figuren 3 a - c illustriert. Figur 3a zeigt nutförmige Einprägungen 17, die sich längs der Strang- bzw. Draht-Oberfläche 15 erstrecken. In Figur 3b ist die Strang-Oberfläche 15 mit einer Vielzahl von lokalen Einkerbungen 18 versehen, die eine erhöhte Ultraschallreflektivität bewirken. Figur 3c sieht eine gerillte Oberflächenstrukturierung 19 längs der Strang- bzw. Draht-Oberfläche 15 vor.

Die aus den einzelnen Ausführungsbeispielen entnehmbaren Einzelmerkmale lassen sich beliebig kombinieren. Auch die jeweils gleichdimensionierten Längen der Drahtabschnitte 8 in den Figuren 2 a bis d lassen sich individuell dimensionieren ohne dabei einen Nachteil in Hinblick auf Ultraschallwellen Reflektivität in Kauf zu nehmen.

Gleichwohl die aus der Draufsichtdarstellung in Figur 2b entnehmbaren Draht-Augen in Projektion auf einem gleichseitigen Dreieck D liegen und so ein mathematisch definiertes bzw. geometrisch bestimmtes Wiedererkennungsmuster darstellen, was sich vor allem für eine autonomen Bildauswertung besonders eignet, können die folgenden Designparameter der in das Implantat 1 eingeprägten Raumform beliebig gewählt werden:
- Durchmesser d der Draht-Augen,
- Länge L der Drahtabschnitte,
- Winkel α, β zwischen den Drahtabschnitten 8, siehe Figuren 2c, d,
- Anzahl der helikalen Wicklungen pro Draht-Auge
- Geometrische Form jedes Draht-Auges: Kreisförmig, elliptisch, oval, n-eckig
- Wicklungssinn der helikalen Wicklungen

### Bezugszeichenliste

- 1: implantierbarer Marker
- 2: Strang bzw. Draht
- 3, 4: feststehendes Auge
- 5: Hohlkanüle
- 6: Mandrin
- 7: distale Öffnung
- 8: Strang-, Draht-Abschnitt
- 9 - 13: feststehendes Auge
- 14: virtuelle Linearachse
- 15: Strang- bzw. Draht-Oberfläche
- 16: nutförmige Einprägung
- 17: lokale Einkerbungen
- 18: gerillte Oberflächenstruktur
- D: gleichseitiges Dreieck
- d: Durchmesser feststehendes Auge
- L: Länge Strang-Draht-Abschnitt
- α, β: Winkel zwischen den Drahtabschnitten

## Patentansprüche

1. Implantierbarer Marker (1), geeignet zur Anordnung innerhalb einer Hohlkanüle sowie zum Markieren eines tierischen oder menschlichen intrakorporalen Gewebebereiches, der wenigstens einen aus biokompatiblen Material gefertigten Strang (2) aufweist, der eine im Wege eines Form-gebenden Verfahrens eingeprägte Raumform besitzt, die der Strang (2) aus einer ihm durch äußeren mechanischen Zwang aufgezwungenen kompaktierten Raumform nach Wegfall des mechanischen Zwangs annimmt, wobei die dem Strang (2) eingeprägte Raumform wenigstens zwei feststehende Strang-Augen (3, 4) umfasst, die jeweils durch wenigstens eine helikale Wicklung des Stranges (2) gebildet sind, über einen Strangabschnitt (8) einstückig miteinander verbunden sind und deren Form und/oder räumliche Relativlage zueinander in der kompaktierten Raumform sowie in der eingeprägten Raumform unterschiedlich sind,
wobei längs des Stranges (2) wenigstens drei feststehende Strang-Augen (9, 10, 11) ausgebildet sind, die sich jeweils durch wenigstens eine einmalige helikale Wicklung zu einem Kreis mit einer helikalen Steigung auszeichnen, bei der sich der Strang im Überlappungsbereich der Wicklung berührt, und die in der kompaktierten Raumform längs einer virtuellen Linearachse (14) seriell hintereinander und überlappungsfrei nebeneinander in einer Hohlkanüle (5) anordenbar sind, die den äußeren mechanischen Zwang auf den Strang (2) auszuüben vermag, und
die wenigstens drei feststehenden Strang-Augen (9, 10, 11) nach Ausschub aus der Hohlkanüle (5) und Wegfall des mechanischen Zwangs in der eingeprägten Raumform eine von der längs der virtuellen Linearachse (14) einnehmbaren seriellen Anordnung räumlich abweichende Anordnung einnehmen.

2. Implantierbarer Marker nach Anspruch 1,
**dadurch gekennzeichnet, dass** der Wicklungssinn der wenigstens einen helikalen Wicklung eines feststehenden Strang-Auges (9, 10, 11) zwischen zwei längs des Stranges (2) angeordneten Strang-Augen (9, 10, 11) unterschiedlich ist.

3. Implantierbarer Marker nach Anspruch 1 oder 2,
**dadurch gekennzeichnet, dass** jedem feststehenden Strang-Auge (9, 10, 11) eine Augenöffnungsebene (E1, E2) zuordenbar ist,
dass die Augenöffnungsebene (E1, E2) wenigstens eines feststehenden Strang-Auges (9, 10, 11) in der eingeprägten Raumform nicht parallel zur Augenöffnungsebene (E1, E2) des wenigstens einen andern Strang-Auges (9, 10, 11) orientiert ist, oder
dass die Augenöffnungsebenen (E1, E2) aller Strang-Augen (9, 10, 11) in der eingeprägten Raumform parallel orientiert sind.

4. Implantierbarer Marker nach einem der Ansprüche 1 bis 3,
**dadurch gekennzeichnet, dass** das biokompatible Material des Stranges (2) aus einem Ultraschallwellen reflektierenden Material besteht

5. Implantierbarer Marker nach einem der Ansprüche 1 bis 4,
**dadurch gekennzeichnet, dass** der Strang (2) eine Strangoberfläche besitzt, an der zumindest bereichsweise eine Ultraschallwellen reflektierende Oberflächenstrukturierung (16, 17, 18) angebracht ist.

6. Implantierbarer Marker nach einem der Ansprüche 1 bis 5,
**dadurch gekennzeichnet, dass** der aus biokompatiblen Material bestehende Strang (2) wenigstens einen Werkstoff mit Formgedächtniseigenschaften aufweist.

7. Implantierbarer Marker nach einem der Ansprüche 1 bis 6,
**dadurch gekennzeichnet, dass** der aus biokompatiblen Material bestehende Strang (2) ein Draht aus einem metallischen Formgedächtnismaterial der nachstehenden Gruppe ist: NiTi ("Nitinol"), NiTiCu, CuZn, CuZnAl oder CuAlNi.

## Claims

1. An implantable marker (1) suitable for arrangement within a hollow cannula and for marking an animal or human intracorporeal tissue region, said marker comprising at least one strand (2) which is made of biocompatible material and which has a three-dimensional shape impressed upon it in the course of a shaping process, the strand (2) adopting this impressed three-dimensional shape after cessation of an external mechanical constraint, said external mechanical constraint having forced the strand to adopt a compacted three-dimensional shape, wherein the three-dimensional shape impressed upon the strand (2) comprises at least two fixed strand eyelets (3, 4), which are each formed by at least one helical winding of the strand (2), are connected to one another in one piece by a strand portion (8), and the shape and/or relative spatial position of which are different in the compacted three-dimensional shape and in the impressed three-dimensional shape,
wherein
at least three fixed strand eyelets (9, 10, 11) are formed along the strand (2), which are each distinguished by at least one single helical winding forming a circle with a helical pitch, in which the strand contacts itself in the overlap region of the winding, and which, in the compacted three-dimensional shape, are arrangeable one behind the other in series along a virtual linear axis (14) and adjacently, without overlap, in a hollow cannula (5), which makes it possible to exert the external mechanical constraint onto the strand (2), and
the at least three fixed strand eyelets (9, 10, 11), following discharge from the hollow cannula (5) and cessation of the mechanical constraint, in the impressed three-dimensional shape adopt an arrangement spatially deviating from the series arrangement adoptable along the virtual linear axis (14).

2. The implantable marker according to claim 1,
**characterised in that** the winding direction of the at least one helical winding of a fixed strand eyelet (9, 10, 11) is different between two strand eyelets (9, 10, 11) arranged along the strand (2).

3. The implantable marker according to claim 1 or 2,
**characterised in that** an eyelet opening plane (E1, E2) is associatable with each fixed strand eyelet (9, 10, 11),
**in that** the eyelet opening plane (E1, E2) of at least one fixed strand eyelet (9, 10, 11) in the impressed three-dimensional shape is oriented non-parallel to the eyelet opening plane (E1, E2) of the at least one other strand eyelet (9, 10, 11), or
**in that** the eyelet opening planes (E1, E2) of all strand eyelets (9, 10, 11) are oriented in parallel in the impressed three-dimensional shape.

4. The implantable marker according to any one of claims 1 to 3,
**characterised in that** the biocompatible material of the strand (2) consists of an ultrasonic-wave-reflecting material.

5. The implantable marker according to any one of claims 1 to 4,
**characterised in that** the strand (2) has a strand surface, to which there is attached, at least in regions, an ultrasonic-wave-reflecting surface structuring (16, 17, 18).

6. The implantable marker according to any one of claims 1 to 5,
**characterised in that** the strand (2) consisting of biocompatible material comprises at least one material with shape-memory properties.

7. The implantable marker according to any one of claims 1 to 6,
**characterised in that** the strand (2) consisting of biocompatible material is a wire made of a metal shape-memory alloy from the following group: NiTi ("nitinol"), NiTiCu, CuZn, CuZnAl or CuAlNi.

## Revendications

1. Marqueur implantable (1), adapté pour être disposé dans une canule creuse et pour marquer une région tissulaire intracorporelle animale ou humaine, qui présente au moins un brin (2) en matériau biocompatible, qui présente une forme spatiale gaufrée lors d'un processus de mise en forme, qui reçoit le brin (2) formé d'une forme spatiale compactée qui lui est appliquée par une force mécanique extérieure après suppression de la force mécanique, la forme spatiale gaufrée sur le brin (2) comprenant au moins deux œillets de brin fixes (3, 4), qui sont respectivement formés par au moins un enroulement hélicoïdal du brin (2), sont reliés à un autre en formant une seule pièce par l'intermédiaire d'une section de brin (8) et dont la forme et/ou la position relative spatiale l'un par rapport sont différentes dans la forme spatiale compactée ainsi que dans la forme spatiale gaufrée,
au moins trois œillets de brin fixes (9, 10, 11) étant formés le long du brin (2), qui se distinguent chacun par au moins un enroulement hélicoïdal unique en un cercle avec une pente hélicoïdale, où le brin touche l'enroulement dans la zone de chevauchement, et qui peuvent être disposés en forme spatiale compactée le long d'un axe linéaire virtuel (14) en série les uns derrière les autres et sans chevauchement dans une canule creuse (5) qui peut exercer la force mécanique externe sur le brin (2), et
les au moins trois œillets de brin fixes (9, 10, 11), après avoir été poussés hors de la canule creuse (5) et après suppression de la force mécanique dans la forme spatiale gaufrée, adoptant une disposition différant spatialement de la disposition en série qui peut être prise le long de l'axe linéaire virtuel (14).

2. Marqueur implantable selon la revendication 1,
**caractérisé en ce que** le sens d'enroulement d'au moins un enroulement hélicoïdal d'un œillet de brin fixe (9, 10, 11) entre deux œillets de brin (9, 10, 11) disposés le long du brin (2) est différent.

3. Marqueur implantable selon la revendication 1 ou 2, **caractérisé en ce qu'**un plan d'ouverture d'œillet (E1, E2) peut être associé à chaque œillet fixe (9, 10, 11), que le plan d'ouverture d'œillet (E1, E2) d'au moins un œillet de brin fixe (9, 10, 11) dans la forme spatiale gaufrée n'est pas orienté parallèlement au plan d'ouverture d'œillet (E1, E2) de l'au moins un autre œillet de brin (9, 10, 11) ou
**que** les plans d'ouverture d'œillets (E1, E2) de tous les œillets de brin (9, 10, 11) sont orientés parallèlement dans la forme spatiale gaufrée.

4. Marqueur implantable selon l'une quelconque des revendications 1 à 3, **caractérisé en ce que** le matériau biocompatible du brin (2) est constitué d'un matériau réfléchissant les ondes ultrasonores

5. Marqueur implantable selon l'une des revendications 1 à 4, **caractérisé en ce que** le brin (2) présente une surface de brin sur laquelle est fixée au moins par zones une structure superficielle (16, 17, 18) réfléchissant les ondes ultrasonores.

6. Marqueur implantable selon l'une quelconque des revendications 1 à 5, **caractérisé en ce que** le brin (2) constitué d'un matériau biocompatible présente au moins un matériau présentant des propriétés de mémoire de forme.

7. Marqueur implantable selon l'une quelconque des revendications 1 à 6, **caractérisé en ce que** le brin (2) constitué d'un matériau biocompatible est un fil composé d'un un matériau métallique à mémoire de forme du groupe suivant : NiTi (« nitinol »), NiTiCu, CuZn, CuZnAl ou CuAlNi.
